# NOUVEAU FASCICULE DE BREVET EUROPEEN

(11) **EP 0 938 892 B2**
(45) Date de publication et mention de la décision concernant l'opposition: **02.02.2005**
(45) Mention de la délivrance du brevet: 06.03.2002
(21) Numéro de dépôt: 99400098.2
(22) Date de dépôt: 15.01.1999
(51) Int. Cl.: A61K 7/48, A61K 7/035

(54) **Poudre cosmétique et/ou dermatologique, son procédé de préparation et ses utilisations**
Kosmetische und/oder dermatologische Pulverzusammensetzung, sowie Verfahren zur Herstellung und ihre Anwendung
Cosmetic and/or dermatological powder, its process for manufacturing and its use.

(30) Priorité: 03.02.1998 FR 9801219
(43) Date de publication de la demande: 01.09.1999
(73) Titulaire: L'OREAL, 75008 Paris (FR)
(72) Inventeur: Roulier, Véronique, 75010 Paris (FR); Bordeaux, Dominique, 91310 Long Pont/s/Orge (FR)
(74) Mandataire: Rasson, Catherine

(56) Documents cités:
- EP-A- 0 659 403
- EP-A- 0 664 112
- US-A- 3 971 852
- US-A- 5 063 050
- CHEMICAL ABSTRACTS, vol. 123, no. 4, 24 juillet 1995 Columbus, Ohio, US; abstract no. 40707, OOYAMA, KEIICHI ET AL: "Powdered oily ingredient-containing compositions and cosmetics and topical preparations containing them" XP002087236 & JP 07 053324 A (NISSHIN OIL MILLS LTD, JAPAN)
- CHEMICAL ABSTRACTS, vol. 125, no. 20, 11 novembre 1996 Columbus, Ohio, US; abstract no. 250830, FUKUDA, HAJIME ET AL: "Starch ester-based compounds for use as powdered carriers for organic liquids" XP002087237 & JP 08 183805 A (NIPPON STARCH REFINING, JAPAN)
- CHEMICAL ABSTRACTS, vol. 127, no. 11, 15 septembre 1997 Columbus, Ohio, US; abstract no. 152818, YAMAMOTO, NAOJI: "Deodorant aerosol compositions containing starch octenylsuccinate and inorganic powders" XP002087238 & JP 09 194341 A (KANEBO, LTD., JAPAN)
- G. PROSERPIO: "Amido-Ottenilsuccinato di Alluminio" COSMETIC AND TOILETRIES, ED. ITALIAN., vol. 4, no. 12, 1991, page 38-43 XP002087703
- CHEMICAL ABSTRACTS, vol. 128, no. 3, 19 janvier 1998 Columbus, Ohio, US; abstract no. 26747, HASEGAWA, YUKO: "Oil-in-water emulsion cosmetics" XP002087239 & JP 09 278644 A (NOEVIR K. K., JAPAN)

## Description

L'invention se rapporte à une poudre cosmétique ou dermatologique, à son procédé de fabrication et à ses utilisations notamment pour le soin, le nettoyage, le démaquillage, le maquillage et/ou le traitement de la peau, des muqueuses et/ou du cuir chevelu.

Il est connu dans le domaine cosmétique ou dermatologique, que pour remédier aux défauts (toucher gras, difficulté de prise) des produits anhydres communément utilisés, on peut utiliser des poudres renfermant un important pourcentage d'huile, ces poudres ayant ainsi l'avantage d'être confortables à l'application. Toutefois, ces poudres contiennent la plupart du temps des tensioactifs, agents potentiels d'irritation, et sont généralement réhydratées au moment de l'utilisation, d'où des difficultés de manipulation.

Par ailleurs, on connaît des poudres de maquillage qui comportent un faible taux d'huile et un fort taux de charges et ne peuvent être utilisées comme produit de soin, car elles présentent alors l'inconvénient de n'être pas suffisamment confortables du fait de leur faible taux de phase huileuse et de laisser nécessairement un film visible à la surface de la peau.

Dans le domaine du soin de la peau, le document EP-A-664112 décrit une poudre à base d'huile, de biopolymères et de polysaccharides, obtenue à partir d'une émulsion huile-dans-eau (H/E) et pouvant être utilisée telle quelle ou après ré-hydratation en une émulsion H/E. Toutefois, une telle poudre présente l'inconvénient de contenir des biopolymères choisis parmi les protéines d'origine animale ou végétale et indispensables à la constitution de la poudre puisqu'ils en constituent le support. Or, on évite d'utiliser dans des produits cosmétiques ou dermatologiques, des protéines d'origlne animale. En outre, ces protéines se conservent mal au cours du temps, et certaines protéines d'origine végétale, telles que les protéines de soja, peuvent se révéler irritantes, notamment lorsqu'elles sont utilisées en une quantité d'au moins 4 %, ce qui est le cas dans le présent document.

Par ailleurs, la poudre obtenue selon ce document est hygroscopique, ce qui est un inconvénient lorsque l'on souhaite utiliser la poudre telle quelle, puisqu'elle a tendance à s'humidifier et présente de ce fait une mauvaise conservation dans les pays tropicaux humides.

Par ailleurs, le document WO-A-95/28849 décrit des compositions solides utilisables dans le domaine alimentaire, préparées par déshydratation de dispersions aqueuses d'amidon et d'huile, pouvant être facilement redispersées dans l'eau en formant des dispersions stables sans nécessiter l'utilisation d'émulsifiant. Le procédé utilisé selon ce document consiste à soumettre le mélange amidon, huile et phase aqueuse à des températures et pressions élevées afin de solubillser complètement l'amidon dans la phase aqueuse. Cette technique présente l'inconvénient de nécessiter l'utilisation de températures élevées lors du mélange de l'amidon, de l'huile et de la phase aqueuse, ces températures élevées particulièrement préjudiciables lorsque la composition contient des huiles d'origine végétale qui sont facilement oxydables et donc sensibles aux températures élevées ou lorsque l'on veut ajouter des actifs sensibles à la température, ce qui est fréquemment le cas dans le domaine cosmétique. De plus, la poudre obtenue est hygroscopique et donc sensible à la présence d'eau, avec les mêmes inconvénients qu'indiqués ci-dessus. En outre, dans les exemples cités dans ce document, l'amidon utilisé est l'amidon natif qui présente, outre l'inconvénient de donner des compositions hygroscopiques, celui d'avoir une mauvaise protection bactériologique.

La demanderesse a trouvé de manière inattendue que l'utilisation d'amidon modifié permettait d'obte nir une poudre cosmétique ou dermatologique anhydre comprenant de l'acide ascorbique pouvant contenir au moins un corps gras, n'ayant pas les inconvénients notamment d'hygroscopie des poudres de l'art antérieur et dont la préparation ne nécessite pas la solubilisation complète de l'amidon et donc l'utilisation de températures et pressions élevées lors du mélange amidon, huile et phase aqueuse.

La présente invention a donc pour objet une poudre cosmétique ou dermatologique, caractérisée en ce qu'elle comprend de 5 à 50 % en poids d'au moins un amidon modifié et de 50 à 95 % en poids d'une phase huileuse comprenant au moins une huile, par rapport au poids total de la composition, le support pondéral phase huileuse/amidon allant de 1 à 19, et en ce qu'elle comprend de l'acide ascorbique.

On entend par "poudre" une substance solide divisée en particules ou grains très fins et homogènes.

La poudre selon l'invention est de préférence exempte de protéine.

La poudre selon l'invention comprend une phase huileuse fixée dans l'amidon modifié.

Cette poudre présente notamment les avantages d'être applicable surtout type de peaux sans laisser d'effet gras malgré la quantité importante d'huile, d'être facile et rapide à utiliser, de pénétrer dans la peau sans laisser de film visible, de conserver les propriétés hydratantes des corps gras et de ne pas nécessiter l'ajout d'un liquide tel que l'eau puisqu'elle peut être utilisée telle quelle. En outre, on peut facilement en prélever la quantité souhaitée.

En outre, du fait que cette poudre peut être obtenue sans émulsionnant et qu'elle se conserve bien, on peut éviter l'ajout d'émulsionnants et/ou de conservateurs, et ainsi obtenir une poudre beaucoup moins irritante que les produits de soin classiques.

Par ailleurs, une telle présentation d'un produit cosmétique permet l'incorporation de composés ayant différentes propriétés physico-chimiques. Ce produit peut ainsi comprendre des détergents. Ceci permet notamment de nettoyer la peau tout en l'hydratant et la nourrissant. On a donc à faire à un nouveau concept de produit cosmétique ou dermatologique d'applications variées, non spécifique à un type de peau,

Par ailleurs, on peut incorporer dans ces poudres, des composés connus pour être sensibles à l'eau et/ou à l'oxydation tels que des vitamines (vitamine A ou ses esters) ou des enzymes. Dans ce milieu, ces composés vont se conserver longtemps et donc ne pas perdre leur activité au cours du temps.

La poudre selon l'invention présente une granulométrie (ou dimension moyenne en nombre de particules) pouvant aller notamment d'environ 0,1 à 100 µm, de préférence de 0,5 à 50 µm et mieux de 1 à 10 µm. La granulométrie est mesurée avec l'appareil : "MI-CROTRAC X100 & SRA 150" de la société LEEDS-NORTHRUP.

L'amidon modifié utilisé dans la composition de l'invention peut être modifié par une ou plusieurs des réactions suivantes : prégélatinisation, oxydation, réticulation, estérification.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymèrisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glyceryl et/ou phosphate) ;
- estérification en milieu alcalin pour le greffage de groupes fonctionnels, notamment acétyl, hydroxyéthyl, hydroxypropyl, carboxyméthyl, octénylsuccinique.

Comme amidons modifiés utilisables selon l'invention, on peut citer par exemple les amidons estérifiés par l'anhydride octénylsuccinique et plus particulièrement le "Aluminium Starch octenyl succinate" tel que le produit vendu par la société NATIONAL STARCH sous le nom de DRY-FLO, l'amidon de maïs réticulé vendu sous le nom RESISTAMYL E2 par la société AMYLUM ; l'amidon de pomme de terre estérifié par un groupe carboxyméthyl vendu sous le nom SUPRAMYL P 60 par la société AMYLUM, l'amidon de maïs estérifié par un groupe hydroxypropyle vendu sous le nom ME-RIGEL EF6 par la société AMYLUM ; l'amidon prégélatimisé, modifié par l'anhydride octénylsuccinique, puis par un motif hydrophobe, vendu sous le nom de NA-TROSORB HFB par la société NATIONAL STARCH ; l'amidon de maïs réticulé et acétylé vendu par la société CERESTAR sous le nom C* Flo 06205.

Selon un mode préféré de réalisation de l'invention, les amidons utilisés sont le DRY-FLO et le C* Flo 06205.

La phase huileuse contient au moins une huile. L'huile utilisée peut être choisie parmi les huiles minérales telles que les huiles de paraffine ou la vaseline, les huiles de silicone telles que les huiles de silicone volatile, les huiles d'origine végétale (par exemple l'huile d'amandes douces, l'huile d'amandes d'abricot), les huiles d'origine animale, les huiles de synthèse et leurs mélanges.

On peut ajouter à l'huile ou aux huiles, des matières grasses telles que les acides gras, les alcools gras, les cires telles que les cires d'origine animale comme la cire d'abeille, les cires de carnauba ou de candellila, les cires minérales comme les cires microcristallines et les cires de synthèse comme les cires de polyéthylène ou de silicone.

La poudre de l'invention peut comprendre aussi un ou plusieurs additifs couramment utilisés dans les domaines cosmétique et dermatologique. Comme additifs, on peut citer par exemple les actifs cosmétiques ou dermatologiques, les émulsionnants ou tensioactifs, les détergents, les matières colorantes y compris les pigments, les abrasifs, les agents antioxydants ou anti-radicaux libres, les charges, les parfums. Ces additifs peuvent représenter de 0 à 30% du poids total de la poudre, de préférence de 0,5 à 15% du poids total de la poudre.

Comme actifs, on peut citer par exemple les agents anti-acné, antimicrobiens, antitranspirants, astringents, déodorants, dépilatoires, analgésiques externes, les agents de conditionnement des cheveux, de conditionnement de la peau, de protection solaire, les vitamines, les acides gras essentiels, les agents kératolytiques, les enzymes, les agents hydratants, les anti-inflammatoires, les détergents ou agents moussants, les parfums, les charges matifiantes minérales ou organiques, les dépigmentants.

De façon avantageuse, la poudre de l'invention comporte un milieu physiologiquement acceptable pour la peau, le cuir chevelu et/ou les muqueuses.

Un autre objet de l'invention concerne un procédé de fabrication d'une poudre, notamment cosmétique ou dermatologique comprenant de l'acide ascorbique, comprenant au moins un amidon modifié et une phase huileuse, le dit procédé comprenant (1) la préparation d'une dispersion huile-dans-eau par mélange d'une phase huileuse comprenant au moins une huile dans une phase aqueuse comprenant au moins un amidon modifié, le rapport pondéral phase huileuse/amidon allant de 1 à 19, et (2) ia déshydratation de ladite dispersion pour obtenir ladite poudre.

Par dispersion, on entend toute dispersion ou émulsion huile-dans-eau, c'est-à-dire tout mélange d'une phase huileuse dans une phase aqueuse en présence ou non d'un émulsionnant.

La phase aqueuse de la dispersion huile-dans-l'eau représente de préférence au moins 30% en poids de la dispersion.

Le rapport pondéral phase huileuse/amidon est de préférence de 1 à 19 et mieux de 2 à 10.

Selon un mode particulier de réalisation du procédé, la dispersion utilisée est telle qu'elle a une teneur en matière sèche comprise entre 5 et 70 % en poids et de préférence comprise entre 10 et 60 % en poids. Une telle teneur en matière sèche permet d'avoir une viscosité de la dispersion, telle qu'elle soit suffisamment fluide pour pouvoir être utilisée pour la suite des étapes.

La phase aqueuse peut être préparée à toute température (0 à 100 °C). On travaille de préférence à une température allant de 80° à 100° C.

De manière parallèle, on prépare la phase huileuse et on l'ajoute à la phase aqueuse qui est de préférence refroidie à une température inférieure à 80°, de préférence en une quantité telle qu'on a, dans la dispersion qui en résulte, une teneur en matière sèche comprise entre 5 et 70 % en poids.

On forme alors la dispersion en mélangeant lentement les phases aqueuse et huileuse, par exemple en incorporant doucement la phase huileuse dans la phase aqueuse tout en maintenant une agitation constante.

On obtient ainsi une dispersion huile-dans-eau, présentant un pH dépendant de sa composition, mais généralement compris entre 4 et 9.

De manière préférée, le procédé comprend une étape d'homogénéisation entre la préparation de la dispersion par mélange de la phase huileuse et de la phase aqueuse et avant la déshydratation de la dispersion obtenue. L'homogénéisation est avantageusement réalisée sous pression élevée, de manière à diminuer la taille moyenne des gouttelettes de la phase huileuse jusqu'à environ 350 nm, voire moins. L'homogénéisation est réalisée sous une pression en général comprise entre 300 et 600 bars, de préférence d'environ 600 bars (60 x 10⁶ Pa), pour obtenir des gouttelettes de phase huileuse ayant une taille moyenne (en nombre) de préférence inférieure à 350 nm, allant généralement de 80 à 300 nm.

La dispersion ainsi homogénéisée est alors déshydratée partout procédé connu, et notamment par atomisation ou par lyophilisation. Selon un mode préféré de réalisation de l'invention, la déshydratation est effectuée par atomisation. Dans ce cas, la température de l'air chaud utilisé pour le séchage va de préférence d'environ 100°C à 220°C, et la température de sortie de la poudre va de préférence d'environ 30°C à 140°C. Le temps d'atomisation est très bref ; il est de préférence inférieur ou égal à 2 minutes.

La présente invention a aussi pour objet une poudre notamment cosmétique et/ou dermatologique susceptible d'être obtenue selon le procédé décrit ci-dessus.

La poudre obtenue peut être compactée pour gagner du volume et pour en faciliter le conditionnement, la conservation, le stockage et l'emploi. Avant le compactage éventuel de la poudre, celle-ci peut subir une étape de granulation supplémentaire ayant pour but d'homogénéiser la granulométrie de la poudre.

La poudre obtenue est très stable et se conserve plusieurs mois sans que l'on puisse observer de séparation de phases, d'évolution de la couleur, de reprise d'eau ou d'autres dégradations, microbiologiques par exemple.

En outre, les actifs sensibles à l'eau ou à l'oxydation, tels que le thé vert ou les enzymes, restent très stables dans ce type de poudre.

La poudre de l'invention peut être utilisée directement telle quelle ou incorporée dans une composition cosmétique et/ou dermatologique se présentant sous une autre forme galénique telle que lotion, émulsion E/H ou H/E.

L'utilisation ultérieure de la poudre de l'invention dépend du but recherché et des actifs éventuellement présents. Elle peut être en particulier utilisée pour le soin, le nettoyage, le démaquillage, le maquillage et/ou le traitement de la peau humaine, du cuir chevelu et/ou des muqueuses.

Aussi, la présente invention a encore pour objet un procédé cosmétique de soin, de nettoyage, de démaquillage, de maquillage et/ou de traitement de la peau, des muqueuses et/ou du cuir chevelu, comprénant l'application sur la peau, les muqueuses et/ou le cuir chevelu, d'une poudre telle que définie précédemment.

Un autre objet de l'invention est l'utilisation de la poudre telle que définie ci-dessus pour la préparation d'une composition destinée au soin, au nettoyage, au démaquillage, au maquillage et/ou au traitement de la peau, du cuir chevelu et/ou des muqueuses.

L'invention est illustrée plus en détail à l'aide de l'exemples qui suit

### Exemple 1: Poudre de soin de la peau - Exemple de référence ne faisant pas partie de l'invention.

- Amidon modifié (DRY FLO)) 25 %
- Huile d'abricot 75 %

Pour obtenir cette poudre, on prépare un mélange contenant 8 % d'amidon modifié et 68 % d'eau et on chauffe le mélange à 95°C, puis on le refroidit à 70 °C avant d'incorporer 24 % d'huile d'abricot, tout en maintenant une agitation constante de manière à obtenir une dispersion huile-dans-eau. On refroidit la dispersion jusqu'à température ambiante tout en l'agitant et on l'homogénéise sous une pression d'environ 600 bars (60 x 10⁶ Pa), puis on la passe dans un appareil d'atomisation où l'air chaud est à 200°C et la température de sortie est de 120°C.

La poudre est utilisée telle quelle, non reconstituée, et constitue un produit efficace pour nourrir la peau.

### Exemple 2 : Poudre de soin de la peau

- Amidon modifié (C⁻ Flo 06205) 35 %
- Huile de silicone (Phényl triméthicone vendu sous le nom DC 556 Fluid par Dow Coming) 60 %
- Acide ascorbique 5 %

Le procédé de fabrication est identique à celui de l'exemple 1, exemple de référence.

On obtient une poudre apte à être appliquée sur la peau pour améliorer l'éclat du teint et lisser les traits.

La vitamine C reste stable dans cette formule : après 2 mois à 45° C, on observe moins de 5% de dégradation.

## Revendications

1. Poudre cosmétique ou dermatologique, **caractérisée en ce qu'**elle comprend de 5 à 50 % en poids d'au moins un amidon modifié et de 50 à 95 % en poids d'une phase huileuse comprenant au moins une huile, par rapport au poids total de la composition, le rapport pondéral phase huileuse/amidon allant de 1 à 19, et **en ce qu'**elle comprend de l'acide ascorbique.

2. Poudre selon la revendication 1, **caractérisée en ce qu'**elle est exempte de protéine.

3. Poudre selon l'une quelconque des revendications précédentes, **caractérisée en ce que** l'amidon est modifié par au moins une réaction choisie parmi la prégélatinisation, l'oxydation, la réticulation et l'estérification.

4. Poudre selon l'une quelconque des revendications précédentes, **caractérisée en ce que** l'amidon modifié est l'amidon estérifié par l'anhydride octénylsuccinique.

5. Poudre selon l'une quelconque des revendications précédentes, **caractérisée en ce que** la phase huileuse comprend au moins une huile choisie parmi les huiles minérales, les huiles de silicone, les huiles d'origine végétale, les huiles d'origine animale et les huiles de synthèse.

6. Poudre selon l'une quelconque des revendications précédentes, **caractérisée en ce que** la phase huileuse contient en outre au moins une matière grasse choisie parmi les acides gras, les alcools gras et les cires.

7. Poudre selon l'une quelconque des revendications précédentes, **caractérisée en ce qu'**elle contient en outre, au moins un additif choisi parmi les actifs cosmétiques ou dermatologiques, les émulsionnants, les détergents, les matières colorantes, les abrasifs, les agents antioxydants ou anti-radicaux libres, les parfums et les charges.

8. Poudre selon la revendication précédente, **caractérisée en ce que** l'actif cosmétique ou dermatologique est choisi parmi les agents anti-acné, antimicrobiens, antitranspirants, astringents, déodorants, dépilatoires, analgésiques externes, les agents de conditionnement des cheveux, de conditionnement de la peau, de protection solaire, les vitamines, les acides gras essentiels, les agents kératolytiques, les enzymes, les agents hydratants, les anti-inflammatoires, les détergents, les parfums, les charges matifiantes minérales ou organiques et les dépigmentants.

9. Poudre selon la revendication 7 ou 8, **caractérisée en ce que** l'actif est choisi parmi le rétinol et ses esters, les enzymes, le thé vert.

10. Procédé de fabrication d'une poudre cosmétique et/ou dermatologique, exempte de protéine, comprenant de l'acide ascorbique et comprenant de 5 à 50 % en poids d'au moins un amidon modifié et de 50 à 95 % en poids d'une phase huileuse, comprenant (1) la préparation d'une dispersion huile-dans-eau par mélange d'une phase huileuse comprenant au moins une huile dans une phase aqueuse comprenant au moins un amidon modifié, le rapport pondéral phase huileuse/amidon allant de 1 à 19, et (2) la déshydratation de ladite dispersion pour obtenir ladite poudre, le dit procédé comprenant une étape d'homogénéisation entre la préparation de la dispersion et avant la déshydratation de la ditedispersion, où l'homogénéisation est réalisée sous une pression comprise entre 300 et 600 bars.

11. Procédé selon la revendication 10, **caractérisé en ce que** la phase aqueuse de la dispersion huile-dans-eau représente au moins 30% du poids total de la dispersion.

12. Procédé selon la revendication précédente, **caractérisé en ce que** l'homogénéisation est réalisée sous une pression d'environ 600 bars.

13. Procédé selon l'une quelconque des revendications 10 à 12, **caractérisé en ce que** la déshydratation est effectuée par atomisation.

14. Procédé selon la revendication précédente, **caractérisé en ce que** la déshydratation est réalisée dans un appareil d'atomisation où la température de l'air chaud utilisé pour le séchage va d'environ 100°C à 220°C, et la température de sortie de la poudre va d'environ 30°C à 140°C.

15. Poudre cosmétique et/ou dermatologique susceptible d'être obtenue par le procédé selon l'une quelconque des revendications 10 à 14.

16. Procédé cosmétique de soin, de nettoyage, de démaquillage, de maquillage et/ou de traitement de la peau, des muqueuses et/ou du cuir chevelu, comprenant l'application sur la peau, les muqueuses et/ou le cuir chevelu, d'une poudre selon l'une quelconque des revendications 1 à 9 et 15, les méthodes de traitement thérapeutiques étant exclues.

17. Utilisation de la poudre selon l'une quelconque des revendications 1 à 9 et 15 pour la préparation d'une composition destinée au soin, au nettoyage, au démaquillage, le maquillage et/ou au traitement de la peau, du cuir chevelu et/ou des muqueuses.

## Claims

1. Cosmetic or dermatological powder, **characterized in that** it comprises from 5 to 50% by weight of at least one modified starch and from 50 to 95% by weight of an oily phase comprising at least one oil, relative to the total weight of the composition, the oily phase/starch weight ratio ranging from 1 to 19, and **in that** it comprises ascorbic acid.

2. Powder according to Claim 1, **characterized in that** it contains no protein.

3. Powder according to either one of the preceding claims, **characterized in that** the starch is modified by at least one reaction chosen from pregelatinization, oxidation, crosslinking and esterification.

4. Powder according to any one of the preceding claims, **characterized in that** the modified starch is starch esterified with octenylsuccinic anhydride.

5. Powder according to any one of the preceding claims, **characterized in that** the oily phase comprises at least one oil chosen from mineral oils, silicone oils, oils of plant origin, oils of animal origin and synthetic oils.

6. Powder according to any one of the preceding claims, **characterized in that** the oily phase also contains at least one fatty substance chosen from fatty acids, fatty alcohols and waxes.

7. Powder according to any one of the preceding claims, **characterized in that** it also contains at least one additive chosen from cosmetic or dermatological active agents, emulsifiers, detergents, dyestuffs, abrasive agents, antioxidants or anti-free radical agents, fragrances and fillers.

8. Powder according to the preceding claim, **characterized in that** the cosmetic or dermatological active agent is chosen from antiacne agents, antimicrobial agents, antiperspirants, astringent agents, deodorants, hair-removing agents, external analgesics, hair conditioners, skin conditioners, antisun agents, vitamins, essential fatty acids, keratolytic agents, enzymes, moisturizers, antiinflammatories, detergents, fragrances, inorganic or organic matt-effect fillers and depigmenting agents.

9. Powder according to Claim 7 or 8, **characterized in that** the active agent is chosen from retinol and its esters, enzymes and green tea.

10. Process for manufacturing a cosmetic and/or dermatological powder free from protein, comprising ascorbic acid and comprising from 5 to 50% by weight of at least one modified starch and from 50 to 95% by weight of an oily phase, comprising (1) the preparation of an oil-in-water dispersion by mixing an oily phase comprising at least one oil in an aqueous phase comprising at least one modified starch, the oily phase/starch weight ratio ranging from 1 to 19, and (2) dehydration of the said dispersion in order to obtain the said powder, the said process comprising a homogenization step between the preparation of the dispersion and before dehydration of the said dispersion, in which the homogenization is carried out at a pressure of between 300 and 600 bar.

11. Process according to Claim 10, **characterized in that** the aqueous phase of the oil-in-water dispersion represents at least 30% of the total weight of the dispersion.

12. Process according to the preceding claim, **characterized in that** the homogenization is carried out at a pressure of about 600 bar.

13. Process according to any one of Claims 10 to 12, **characterized in that** the dehydration is carried out by spraying.

14. Process according to the preceding claim, **characterized in that** the dehydration is carried out in a spraying machine in which the temperature of the hot air used for the drying ranges from about 100°C to 220°C, and the outlet temperature of the powder ranges from about 30°C to 140°C.

15. Cosmetic and/or dermatological powder which can be obtained by the process according to any one of Claims 10 to 14.

16. Cosmetic process to care for, cleanse, remove make-up from, make up and/or treat the skin, mucous membranes and/or the scalp, comprising the application of a powder according to any one of Claims 1 to 9 and 15 to the skin, mucous membranes and/or the scalp, therapeutic treatment methods being excluded.

17. Use of the powder according to any one of Claims 1 to 9 and 15 for the preparation of a composition intended to care for, cleanse, remove make-up from, make up and/or treat the skin, the scalp and/or mucous membranes.

## Patentansprüche

1. Kosmetisches oder dermatologisches Pulver, **dadurch gekennzeichnet, daß** es 5 bis 50 Gew.-% mindestens einer modifizierten Stärke und 50 bis 95 Gew.-% Ölphase, die mindestens ein Öl enthält, bezogen auf das Gesamtgewicht der Zusammensetzung, enthält, wobei das Gewichtsverhältnis von Ölphase/Stärke im Bereich von 1 bis 19 liegt, und dadurch, daß es Ascorbinsäure enthält.

2. Pulver nach Anspruch 1, **dadurch gekennzeichnet, daß** es keine Proteine enthält.

3. Pulver nach einem der vorhergehenden Ansprüche, **dadurch gekennzeichnet, daß** die Stärke durch mindestens eine Reaktion modifiziert wurde, die unter Verkleisterung, Oxidation, Vernetzung und Veresterung ausgewählt ist.

4. Pulver nach einem der vorhergehenden Ansprüche, **dadurch gekennzeichnet, daß** es sich bei der modifizierten Stärke um eine mit Octenylbernsteinsäureanhydrid veresterte Stärke handelt.

5. Pulver nach einem der vorhergehenden Ansprüche, **dadurch gekennzeichnet, daß** die Ölphase mindestens ein Öl enthält, das unter den Mineralölen, Siliconölen, Ölen pflanzlicher Herkunft, Ölen tierischer Herkunft und den synthetischen Ölen ausgewählt ist.

6. Pulver nach einem der vorhergehenden Ansprüche, **dadurch gekennzeichnet, daß** die Ölphase ferner mindestens eine Fettsubstanz enthält, die unter den Fettsäuren, Fettalkoholen und Wachsen ausgewählt ist.

7. Pulver nach einem der vorhergehenden Ansprüche, **dadurch gekennzeichnet, daß** es ferner mindestens einen Zusatzstoff enthält, der unter den kosmetischen oder dermatologischen Wirkstoffen, Emulgatoren, reinigenden Stoffen, Farbmitteln, abrasiven Mitteln, Antioxidantien, Radikalfängern für freie Radikale, Parfums und Füllstoffen ausgewählt ist.

8. Pulver nach dem vorhergehenden Anspruch, **dadurch gekennzeichnet, daß** der kosmetische oder dermatologische Wirkstoff unter den Wirkstoffen gegen Akne, antimikrobiellen Mittel, Antitranspirantien, Adstringentien, Deodorantien, Enthaarungsmitteln, äußerlich anzuwenden Analgetika, Mitteln zur Konditionierung der Haare, Mitteln zur Konditionierung der Haut, Sonnenschutzmitteln, Vitaminen, essentiellen Fettsäuren, Keratolytika, Enzymen, Hydratisierungsmitteln, entzündungshemmenden Wirkstoffen, Detergentien, Parfums, anorganischen oder organischen mattierenden Füllstoffen und Depigmentierungsmitteln ausgewählt ist.

9. Pulver nach Anspruch 7 oder 8, **dadurch gekennzeichnet, daß** der Wirkstoff unter Retinol und seinen Estern, Enzymen und grünem Tee ausgewählt ist.

10. Verfahren zur Herstellung eines kosmetischen und/oder dermatologischen Pulvers, das frei von Proteinen ist und Ascorbinsäure und 5 bis 50 Gew.-% mindestens einer modifizierten Stärke und 50 bis 95 % Ölphase enthält, das (1) die Herstellung einer Öl-in-Wasser-Dispersion durch Mischen einer Ölphase, die mindestens ein Öl enthält, und einer wäßrigen Phase, die mindestens eine modifizierte Stärke enthält, wobei das Gewichtsverhältnis von Ölphase / Stärke im Bereich von 1 bis 19 liegt, und (2) die Dehydratisierung der Dispersion zur Herstellung eines Pulvers umfaßt, wobei das Verfahren nach der Herstellung der Dispersion und vor der Dehydratisierung der Dispersion einen Homogenisierungsschritt umfaßt, wobei die Homogenisierung bei einem Druck von 300 bis 600 bar erfolgt.

11. Verfahren nach Anspruch 10, **dadurch gekennzeichnet, daß** die wäßrige Phase der Öl-in-Wasser-Dispersion mindestens 30 % des Gesamtgewichts der Dispersion ausmacht.

12. Verfahren nach dem vorhergehenden Anspruch, **dadurch gekennzeichnet, daß** die Homogenisierung bei einem Druck von etwa 600 bar erfolgt.

13. Verfahren nach einem der Ansprüche 10 bis 12, **dadurch gekennzeichnet, daß** die Dehydratisierung durch Zerstäuben durchgeführt wird.

14. Verfahren nach dem vorhergehenden Anspruch, **dadurch gekennzeichnet, daß** die Dehydratisierung in einer Vorrichtung zum Zerstäuben durchgeführt wird, in der die Temperatur der zum Trocknen verwendeten Heißluft im Bereich von etwa 100 bis 220 °C und die Temperatur des Pulvers am Auslaß im Bereich von etwa 30 bis 140 °C liegt.

15. Kosmetisches und/oder dermatologisches Pulver, das nach dem Verfahren nach einem der Ansprüche 10 bis 14 erhältlich ist.

16. Kosmetisches Verfahren zur Pflege, zur Reinigung, zum Abschminken, zum Schminken und/oder zur Behandlung der Haut, der Schleimhäute und/oder der Kopfhaut, das das Aufbringen eines Pulvers nach einem der Ansprüche 1 bis 9 und 15 auf die Haut, die Schleimhäute und/oder die Kopfhaut umfaßt, wobei therapeutische Verfahren ausgenommen sind.

17. Verwendung des Pulvers nach einem der Ansprüche 1 bis 9 und 15 zur Herstellung einer Zusammensetzung zur Pflege, zur Reinigung, zum Abschminken, zum Schminken und/oder zur Behandlung der Haut, der Kopfhaut und/oder der Schleimhäute.
